# EUROPEAN PATENT APPLICATION

(11) **EP 2 359 812 A1**
(43) Date of publication of application: **24.08.2011**
(21) Application number: 10305053.0
(22) Date of filing: 18.01.2010
(51) Int. Cl.: A61K 9/19, A61K 31/05, A61K 31/216, A61K 31/4545, A61K 31/54, A61K 9/20

(54) **Oral lyophilised compositions**

(71) Applicant: CEPHALON FRANCE, 94704 Maisons-Alfort Cedex (FR)
(72) Inventor: Nguyen, Thanh-Tam, 94450, Limeil-Brevannes (FR)
(74) Representative: Colombet, Alain André

(57) **Abstract**

The present invention relates to a method for preparing an oral lyophilizate composition comprising:
a) forming a liquid phase by using at least one homogenising agent having tensioactive properties, said liquid phase comprising at least an active pharmaceutical ingredient, a filler and/or a binding agent and a solvent,
b) lyophilizing said liquid phase to form the oral lyophilizate composition.

## Description

### TECHNICAL FIELD

The present disclosure relates generally to a method for preparing an oral lyophilizate composition comprising the steps of forming a liquid phase by using at least one homogenising agent having tensioactive properties, said liquid phase comprising at least an active pharmaceutical ingredient, a filler and/or a binding agent and a solvent, and then lyophilizing said liquid phase to form an oral lyophilizate composition.

More particularly, the present disclosure pertains to the use of such homogenising agents for the manufacture of oral lyophilized compositions.

The resulting oral lyophilized compositions may have one or more of the following features: a smooth surface, a homogenised colouration, a uniform appearance, and a reduced adherence to packaging during removal. The present disclosure also pertains to processes for obtaining the described oral lyophilized compositions.

### BACKGROUND

Oral lyophilizates are porous, light and stable pharmaceutical solid dosage forms obtained by a lyophilization process. These dosage forms are convenient because they are easy to use without water, as the porous structure of the pharmaceutical form imparted by the freeze-drying process permits rapid disintegration (e.g., breakdown) in water or saliva. A drug product in the form of an oral lyophilizate may preserve many properties of an original liquid drug formulation, in particular mobility and therapeutic activity. Such a method of easy and safe administration combined with a rapid onset of action and a controlled release of the active pharmaceutical ingredient as compared to other classical solid dosage forms can result in a better bioavailability, a better tolerance and an improved patient compliance.

Lyophilized dosage forms are described in e.g., Patent No. U.S 3,855,712, Patent No. US 4,616,047 and Patent US 5,843,347. These lyophilized dosage forms can be based on lyophilized emulsions such as that described in the '047 patent, and can include various diluents, sweeteners and artificial sweeteners, as well as natural or synthetic flavourings and mixtures thereof. See also U.S. Patent N° 4,490,407. Such products are capable of rapidly and integrally dispersing or dissolving in water.

Lyophilization is thought to confer a number of advantages, in particular the preservation of the initial characteristics of the active pharmaceutical ingredient (API), an improvement in its chemical stability (e.g., by avoiding hydrolysis and oxidation reactions), and an improvement in its physical stability by maintaining certain of the characteristics of the active pharmaceutical ingredient during its manufacture (e.g., micronization state, organoleptic properties, crystallinity, and surface treatment of particles). Furthermore, the porous structure of the lyophilizates may prevent the particles from agglomerating when dispersed in water.

Lyophilizates are also interesting as they can often be formulated with high quantities of active pharmaceutical ingredient and, with the exception of some active agents (like proteins) that may be sensitive to heat fluctuations, they are convenient for almost all kinds of active pharmaceutical ingredients, whatever their physico-chemical properties. In particular, they may be well adapted for slightly soluble or insoluble active pharmaceutical ingredients. Moreover, these forms can allow the easy combination of two or more active pharmaceutical ingredients within the same drug unit.

Lyophilization is a multi-stage process which requires a first freezing step to turn a liquid composition into a solid form, then a sublimation step, by which water is removed from the composition under low pressure conditions, which allows water to pass directly from a solid form to a gas form.

Depending on the type of material used to cool down the liquid phase during the first freezing step, the freezing temperature applied to the liquid emulsion may be generally between ―20°C and ―50°C, when performed with conventional mechanical devices (e.g.,classical lyophilizers that use compressor systems like those provided by companies such as Usifroid, Virtis or BOC Edwards, for example), and between -50°C to ― 140°C when liquid gases, in particular liquid nitrogen, are used to freeze the liquid phase.

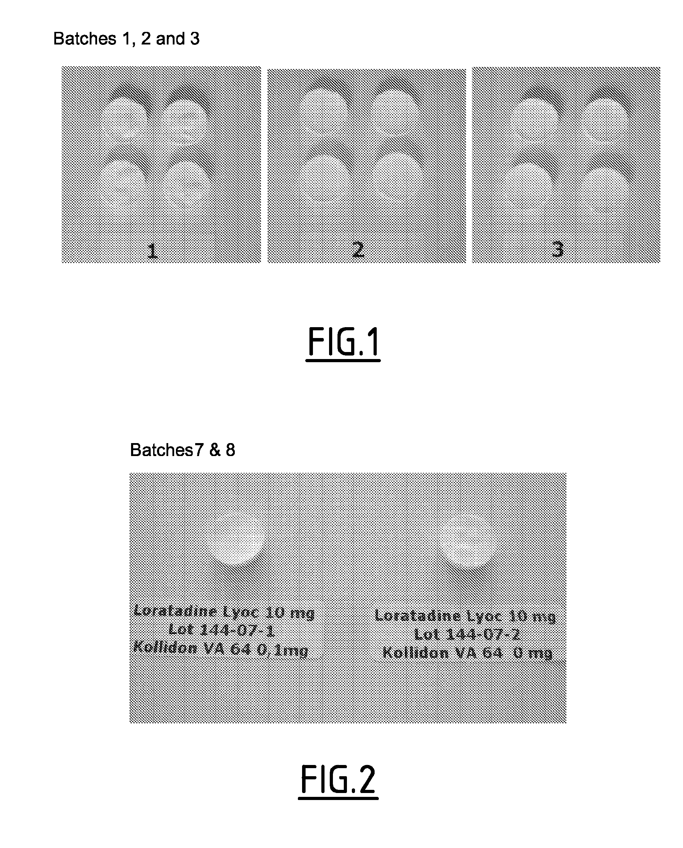

When the freezing phase is performed through mechanical devices, the time duration of a freezing phase at between about -20 to about -50°C is between about 30 and about 90 minutes, depending on the product. During such a relatively long freezing time, it has been observed that the congealing of the liquid phase (under the form of either an emulsion, a solution, or a dispersion) appears to be progressive and non-uniform. Indeed, the freezing begins first at the bottom of the blister (or mould), the water being turned into relatively big crystals, while the top of the blister is still in liquid form. The formation of the ice crystals at the bottom of the blister may be then responsible for a progressive expansion of the volume of the liquid phase from the bottom to the top, pushing up the remaining liquid part of the liquid phase, with the result being that this last liquid part turns into solid when it is still in the shape of a small dome (see photographs of batches 1 and 8 on Figures 1 and 2 respectively). Thus, such a differential freezing step may be responsible for a non uniform appearance of the final lyophilized product.

Others have tried to optimize lyophilization production costs by reducing the lyophilization cycle duration. Such conditions often lead to thermal shocks for the products (e.g., the temperature generally varies rapidly from -50°C to +70°C). These high rates freeze-drying processes create high surface tension, again followed by an increase of the volume of the frozen product. This physical change may have no impact on the quality of the final product, but its final appearance is non uniform and unsightly, which is a drawback from a marketing point of view, as the consumer might think this phenomenon relates to a lack of quality.

In order to reduce the time of the lyophilization cycle, others have tried to heat the shelves supporting the moulds (e.g.: blisters) receiving the liquid composition during the sublimation phase, e.g., to 70° - 80°C, and/or heat the product to 60°C - 65°C. Such a quick change of temperature may cause the lyophilized products to stick to the packaging (e.g., blister packaging), resulting in a fragile and friable product when unpacked.

Other freeze-drying methods with even more drastic conditions have been employed, where very low temperatures are employed to cool down the liquid phase into a solid phase. In these methods, liquid gases like nitrogen are employed during the congealing phase, with the freezing of the emulsion occurring almost instantaneously. Indeed, the liquid phase is poured into the mould/ blisters which have previously been cooled to a very low temperature (between -100 and -140°C) by contact with liquefied gases. The freezing step is then almost instantaneous, preventing the central dome observed with a progressive freezing process to appear. However, another appearance flaw can occur when using this technique. As the freezing step occurs very quickly, it is often observed that the surface of the lyophilizate is altered because the emulsion turns into solid immediately, *i.e.,* even before the surface of the liquid poured into the blister had time to recover a flat surface.

Such visual flaws, even if they do not change the pharmacological profile of the active pharmaceutical ingredient, render lyophilized products unsightly for the patient. For example, it is thought that the aesthetic aspect of a drug product is often, in patients' minds, directly linked to its pharmacological efficiency. This phenomenon can negatively impact the compliance of patients for such a kind of treatment. Thus there is a real need for improving the appearance of lyophilized drug products.

Moreover, it is also important to manufacture lyophilized dosage forms, and in particular tablets, that have a uniform surface, as this renders possible the stamping of the dosage forms for identification purposes. Indeed, it is often required that each individual pharmaceutical form be stamped or printed. Thus, the smoother the surface of the lyophilized forms, the easier may be the printing process and the better the results of such printing/ stamping.

### SUMMARY

The present disclosure pertains to methods for improving certain appearance and structural properties of oral lyophilizates. The methods described herein can improve the visual appearance of oral lyophilizates, including the smoothness of their surface(s), the homogeneity of their colour, and their anti-adhesive properties, while nonetheless preserving or improving their hardness and cohesion. These visual and structural improvements of the lyophilized dosage forms have been discovered to result after the addition of certain specific excipients having tensioactive properties to the dosage forms during manufacturing. These excipients are referred to in the following specification as *"homogenising agents".*

The lyophilized pharmaceutical dosage forms described herein may also be referred to as freeze-dried dosage forms, and are typically obtained by a sublimation step of a solvent from a liquid phase preparation containing a pharmaceutical active ingredient (API).

### BRIEF DESCRIPTION OF THE DRAWINGS

**Figure 1** represents the photographs of tablets coming from batches 1, 2 and 3 obtained according to example 1. The homogenising agent used was caprylocapryl-macrogol 8 glycerides. In batch 1, no homogenising agent was included while in batches 2 and 3, the amount of homogenising agent represented respectively 0.14 and 0.7% of the total dry weight of the composition. The amounts of each excipient are presented in table 1.
**Figure 2****:** represents the photographs of tablets coming from batches 7 and 8 obtained according to example 3. Batch 8 does not contain any homogenising agent while batch 7 contain a copolymer of polyvinylpyrrolidone and polyvinylvinylacetate, also called copovidone, used as a homogenising agent according to the present disclosure.

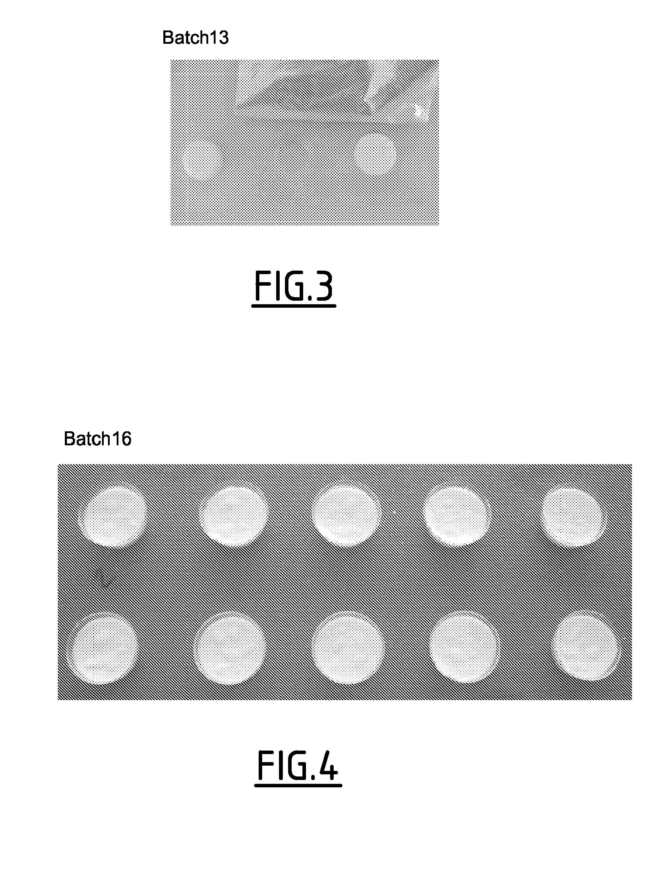

**Figure 3****:** represents the photograph of tablets coming from batch 13 obtained according to example 5. The homogenising agent according to the disclosure is Polysorbate 80 (polyoxyethylene (80) sorbitan monooleate), a sugar ester derivative.
**Figure 4****:** represents the photograph of tablets coming from batch 16 obtained according to example 6. Batch 16 tablets do not contain any homogenising agent according to the method of the present disclosure.

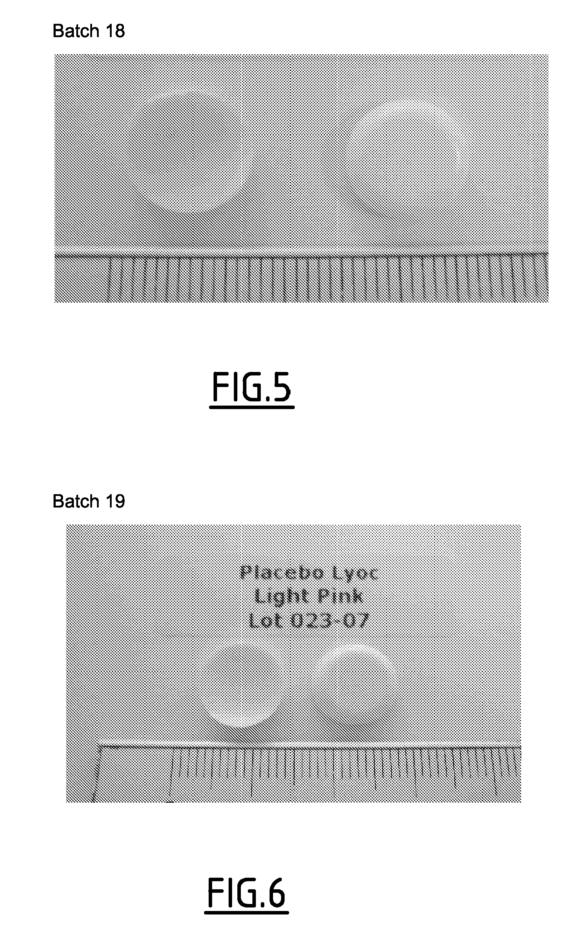

**Figure 5****:** represents the photograph of tablets coming from batch 18 obtained according to example 6. Batch 18 tablets do contain 0.4% Methylcellulose (Methyl ether of cellulose) used as a homogenising agent according to the method of the present disclosure.
**Figure 6****:** represents the photograph of tablets coming from batch 19 obtained according to example 7. The homogenising agent employed in batch 19 was an ether of cellulose (cellulose methyl ether) at a concentration of 1 mg/unit. Batch 19 also contains traces of Erythrosine used as a colouring agent.

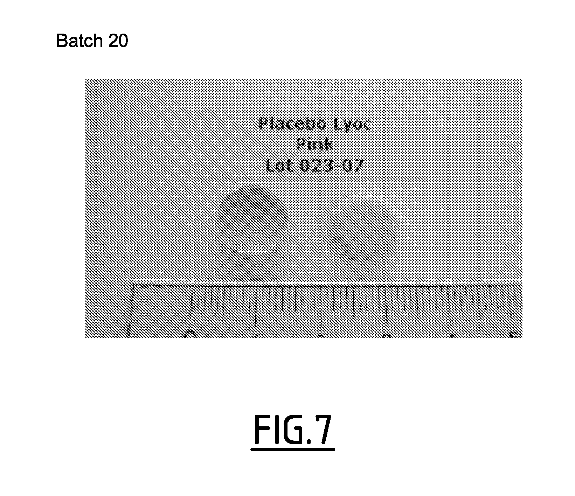

**Figure 7****:** represents the photograph of tablets coming from batch 20 obtained according to example 7. The homogenising agent employed in batch 20 was an ether of cellulose (cellulose methyl ether) at a concentration of 1 mg/unit. Batch 20 also contains traces of Methylene blue used as a colouring agent.

### DETAILED DESCRIPTION

The present invention pertains to a method for preparing an oral lyophilizate composition comprising the steps of forming a liquid phase by using at least one homogenising agent having tensioactive properties, said liquid phase comprising at least an active pharmaceutical ingredient, a filler and/or a binding agent and a solvent and then lyophilizing said liquid phase to form the oral lyophilizate composition.

The method of the invention allows obtaining oral lyophilizate compositions having an improved appearance relative to control oral lyophilizate compositions which does not comprise the homogenising agent.

The present invention also pertains to a process for the manufacture of oral lyophilizate compositions comprising the steps of (i) preparing a liquid phase containing at least the active(s) pharmaceutical ingredient(s), a filler an/or a binding agent and a homogenising agent, (ii) stirring the liquid phase until a homogenous mixture is obtained, (iii) distributing the liquid preparation into preformed blisters or moulds, (iv) freezing the resulting mixture between -20° to - 50°C and (v) freeze-drying the congealed mixture.

It is also an object of the invention an oral lyophilizate composition having an improved appearance relative to a control oral lyophilizate composition which does not comprise homogenising agent, comprising at least an active pharmaceutical ingredient, a filler and/ or a binding agent and a homogenising agent, where the homogenising agent is selected from the group consisting of copolymers of polyvinylpyrrolidone and polyvinylvinylacetate, sucrose esters, methylcellulose or hydroxylpropyl methylcellulose polymers, mixtures of mono, di and triglycerides and mono or diesters of poly ethylene glycol, polyethylene glycol derivatives having a PEG chain with a molecular weight varying from about 100 g/mol to about 1000g/mol, and mixtures of any of the foregoing.

It is also an object of the invention an oral lyophilizate composition having an improved appearance relative to a control oral lyophilizate which does not comprise the homogenising agent comprising: an active pharmaceutical ingredient, a filler and/or a binding agent and a homogenising agent, where the homogenising agent is selected from the group consisting of a copolymer of 1-vinyl-2- pyrrolidone and vinyl acetate in a ratio of 6:4 by mass, sucrose distearate, mixtures of sucrose mono and distearate, sucrose palmitate, polyglycol (300) isostearate, polyethylene 660, 12 hydroxystearate, oleoryl macrogol 6 glucosides, glyceryl palmitostearate, medium chain triglycerides, propylene glycol dicaprylocaprate, caprylocapryl-macrogol 8 glycerides, methylcellulose polymer having from 27.5% to 31.5% of methoxyl groups and hydroxylpropyl methylcellulose polymer having methoxyl content from 19% to 30% and hydroxypropyl content of 7% to 12%, and mixtures of any of the foregoing.

### Definitions

As used herein, the term "active pharmaceutical ingredient" may be used interchangeably with the term drug, API, active agent, active principle, or active ingredient.

The term *"appearance"* will be employed in the present disclosure to refer to the visual and aesthetic aspects of a final drug product. Appearance may be, depending on the product, evaluated based on one or more features, including the regularity, smoothness, and homogeneity of the surfaces and/or the edges, and/or based on the homogeneity of the colour of the lyophilized dosage form. In the present disclosure, the term *"improved appearance"* is employed to qualify the visual appearance of the oral lyophilizate compositions obtained according to the invention, compared to control oral lyophilizate compositions which do not comprise a homogenising agent.

In the present disclosure, the term *"homogenising agent(s)"* is employed to refer to the one or more excipients that are included to improve, e.g., the uniformity, homogeneity and non-adhesive properties of the compositions.

In the present disclosure the terms *"oral lyophilizate"* or *"lyophilizate"* will be used as equivalent terms of *"lyophilized"* or *"freeze-dried"* e.g., with respect to an oral tablet, dosage form, or composition.

In the present disclosure, the term *"tensioactive properties"* is used to describe molecules which have amphiphilic properties, *i.e.* those that include at least one moiety having an affinity for water and more generally for polar compounds, and at least one moiety having an affinity for hydrophobic or non polar or apolar compounds. These compounds thus have both hydrophobic and hydrophilic properties.

The *"HLB",* acronym used to design the Hydrophilic-Lipophilic Balance of a surfactant is a measure of the degree to which it is hydrophilic or lipophilic. It is determined by calculating values for the different regions of the molecule, giving a result on an arbitrary scale of 0 to 20. An HLB value of 0 corresponds to a completely hydrophobic molecule, and a value of 20 would correspond to a molecule made up completely of hydrophilic components. In the present disclosure, the terms "high HLB" will be employed to describe surfactants having a HLB comprised between 15 and 20.

The use of excipients having tensioactive properties within lyophilized formulations has previously been described, but these tensioactive agents had been employed for their usual and known properties of amphiphilicity , e.g., their ability to increase the uniformity of mixtures composed of hydrophobic (e.g., Active Pharmaceutical Ingredients (API)) and hydrophilic agents (e.g., water) or the solubility of hydrophobic compounds within hydrophilic solvents.

For example, Blonde et al. in US 3,855,712, describes lyophilized formulations obtained from an organic liquid phase and the use of polymeric binders which are soluble in water or are capable of giving colloidal solutions in water, but which are also soluble in readily evaporable organic solvents. Such excipients are for example polyvinylpyrrolidone, copolymers of polyvinylpyrrolidone and polyvinyl alcohols, polyvinyl alcohols, carboxypolymethylenes or polyethyleneglycols. These excipients which are chosen because of their particular ability to be soluble both in the organic solvents and water are employed in order to increase the mechanical resistance of the final tablet.

Other excipients, among which are some conventional surfactants, that have tensioactive properties have been employed in, lyophilized formulations, but once again for their usual and known qualities. In patent US 5,827,541, the use of Poloxamers and polysorbates are mentioned as possible surfactants in formulations. The choice of these excipients was made in this patent to enhance the uniformity of the dosage form by preventing hydrophobic active pharmaceutical ingredients, especially when present in the form of microparticles to be badly mixed with the other constituents when suspension is prepared before lyophilization. These agents are also used to prevent the formation of foam on the surface of the suspension.

In US patent US 6,010,719, lyophilized formulations containing at least one surfactant such as polysorbate, lecithins or PEG derivatives are disclosed. In these formulations, the surfactants are used for their usual qualities, e.g., for improving the solubility and enhancing the dispersion of poorly soluble active pharmaceutical ingredients by decreasing the surface tension between the active pharmaceutical ingredient and the surrounding water or hydrophilic medium.

Therefore, these documents do not teach the use of such surfactants in order to improve the physical appearance of these lyophilized products.

Other attempts have been carried out to enhance the physical appearance of lyophilized pharmaceutical products, either in terms of homogeneity, firmness, coloration or conservation of the original shape, in particular by reducing shrinkage.

In patent US 4,537,883, Remon et al. describe a lyophilized formulation of cyclophosphamide, wherein the use of mannitol is responsible for the enhancement of the appearance of the final product. Thereafter, these mannitol-based compositions were revealed to be heat sensitive and to have a poor stability upon storage. To overcome these drawbacks, the disclosed compositions needed to be humidified after lyophilization so that to conserve both good dissolution characteristics and stability upon storage. This further step rendered the manufacturing process longer and more complex to achieve. Moreover, in such compositions, mannitol represents a significant percentage of the final product (until 85 parts by weight versus not more than 25 parts by weight of active pharmaceutical ingredient as described herein. This high amount of mannitol prevents the use of highly concentrated dosage forms, where a high amount of active ingredient would necessitate large amounts of mannitol, resulting in a final tablet of unacceptable size.

In another approach using compositions containing very low quantities of sugar, patent US 5,874,408 describes lyophilized formulations of a protein (recombinant factor Vlll) containing salts and having an acceptable appearance. The authors show that, while improving the stability of the formulation, the use of a significant amount of salt may have a negative impact on the physical appearance of the final product.

In addition, other currently existing lyophilized formulations present some adherence problems with the blister/mould in which they are poured for packaging. Indeed, by sticking to their support, these compositions are often damaged when extracted by the patient, which further degrades the final appearance of the drug product before administration and could contribute to the administration of an incorrect dosage of the drug.

Thus, there is a need for freeze-dried formulations having an improved aesthetic appearance which maintain other qualities such as an acceptable hardness, an acceptable cohesion and homogeneity after extraction from the blister, a good stability upon storage, a high dispersion speed, and a good dissolution profile.

The present disclosure provides a solution to the previously described appearance issues, whatever the method employed to manufacture such dosage forms. It has surprisingly been found that the use of certain excipients having tensioactive properties within such compositions minimizes the appearance flaws described above. Such excipients, referred to as *"homogenising agents"* in the description of the present disclosure, are agents that demonstrate tensioactive properties, as described previously.

Thus, the present disclosure relates to a method for preparing oral lyophilizate compositions in which the use of homogenising agents having tensioactive properties allows the improvement of the appearance of such lyophilizate compositions, in particular oral tablets. The disclosure also covers the processes to manufacture such products and the solid oral lyophilized forms obtained there from. The present disclosure renders possible the manufacture of freeze-dried solid oral dosage forms exhibiting a uniform and smooth surface along with a low adherence to the package within which it is manufactured. Moreover, it also allows the manufacture of coloured lyophilized drug products, wherein the colouring agents can be uniformly distributed, even if they are present in a very small amount. Finally, it also allows or improves the printing, marking or stamping on the surface of each lyophilized unit, rendering possible or more efficient the physical identification of such units.

The lyophilized solid dosage form of the present disclosure comprises at least one active pharmaceutical ingredient (API), at least one homogenising agent, one binding agent and/or one filler.

The present disclosure also covers a process for the manufacture of an oral lyophilizate having an improved appearance relative to a control oral lyophilizate which does not comprise the homogenising agent comprising the steps of:
a. Preparing a liquid phase containing at least the active(s) pharmaceutical ingredient(s), a filler and or a binding agent and a homogenising agent;
b. Stirring the liquid phase until a homogenous mixture is obtained;
c. Distributing the homogeneous mixture into a preformed cavity, (e.g., a blister or mould);
d. Freezing the resulting distributed homogenous mixtures at a temperature from about -20° to about -50°C to result in a frozen congealed mixture;
e. Freeze-drying the frozen congealed mixture; and
f. Optionally performing a secondary drying of the freeze-dried mixture so obtained.

The process of preparation of the compositions according to the present disclosure is based on the common manufacturing process of lyophilizates.

In the first step, a liquid phase to be lyophilized containing the active(s) pharmaceutical ingredient(s) is prepared. Because of the regulatory constraints weighing on pharmaceutical products with respect to the use of organic solvents, the most preferred solvent for the preparation of the liquid phase is water. However, the use of certain organic solvents with a low toxic profile can also be envisaged.

Depending on the physico-chemical characteristics of the active pharmaceutical ingredient (or the eventual mixtures of different active pharmaceutical ingredients) and of its initial form, structure and granulometry, a solution, suspension, emulsion or icy "sherbet-like" preparation can be prepared prior to lyophilization. Such a liquid phase of the present disclosure contains at least the active ingredient, the homogenising agent, the filler and or the binder and water.

Advantageously, other excipients can be added to this liquid phase, in particular, but not limited to: colorants, sweetening agents, taste-masking agents or preservatives.

The mixing of the active pharmaceutical ingredient and appropriate excipients with the exception of the homogenising agent is generally carried out in a mixer equipped with a vacuum system. When in solid form, the active pharmaceutical ingredient and the excipients under powder form are mixed together until an acceptable homogenous mixture is obtained. If active pharmaceutical ingredient is under liquid form it is mixed similarly with the above mentioned excipients which can be either in solid or liquid form.

Generally this mixing step lasts from about 5 to about 30 minutes at reduced pressure (generally from 100 to 300 HPa) in order to degas the powder by aspiration by means of vacuum.

Then, the liquid phase is completed by dispersing the homogenising agent into an pharmaceutically acceptable solvent, preferably water. The resulting solution dispersion or suspension being then added to the mix previously prepared containing the active pharmaceutical ingredient (either in solid or liquid form) and stirred under reduced pressure (generally from 100 to 300 HPa) during a period of from about 30 to about 90 minutes until a homogeneous suspension is obtained.

The quantity of water introduced to form the liquid phase to be freeze-dried is determined so that the final liquid phase has convenient rheological characteristics, i.e. presents a good flow and is easily mixable to a homogenous phase and allows a uniform division of the product when the global volume of liquid phase is divided into individual blisters or moulds. In most cases the quantity of water will be adjusted so that the solid mass constitutes approximately from about 30 to about 80% of the mixture. In some embodiments, it may vary from about 40 to about 60% relative to the dry mass of the mixture.

In a second step, when completely homogenous, the liquid phase is distributed within cavities, generally under the form of thermoformed moulds or blisters made of PVC, PVDC, Aclar or Aluminium foils. The distribution phase is performed mechanically, the global volume of liquid phase being divided into unitary doses having a predetermined shape, size and volume.

The quantity of active pharmaceutical ingredient(s) in the liquid phase, and the shape and the size of the cavities are calculated so as to obtain a precisely defined quantity of the active pharmaceutical ingredient(s) in each unitary dose.

The cavities (i.e.: the blisters) containing the product are placed onto the shelves or between the shelves of the lyophilizer. The shelves are metallic supports for the blisters, that can easily be heated or cooled down and which allow a quick modification of the temperature of the product contained in the blisters.

Once the cavities are filled with the appropriate volume of liquid phase, the freezing or congealing phase of the preparation starts. It begins at a very low temperature (generally between about -20°C to about -50°C), below the freezing point of the liquid phase under atmospheric pressure for about 20 to about 90 minutes until a solid state is obtained. At the end of this step, the active molecules are immobilized, their properties remaining unaltered as the rate of chemical reaction is nearly zero at this low temperature.

The conditions of temperature and pressure of the congealing phase are adapted with respect to the composition of the liquid phase which may be, according to the nature and concentration of the filler and /or binder and homogenising agent, more or less easy to freeze.

The congealed mixture is then subject to sublimation (or freeze-drying), where the product is dried under vacuum by providing simultaneously in a controlled way both heat (and low pressure, generally between about 100 and about 600 µbars, more preferably between about 150 to about 400 µbars. The temperature of the shelves supporting the moulds (or blisters) is increased rapidly from around about -30/-50°C to around + 40/70°C while the temperature of the product increases slowly from the freezing point to +30/+50°C. In these conditions, ice is converted directly into vapour phase. The resulting product is a solid porous dosage form that has conserved its initial shape.

Optionally, an additional drying step (also called secondary drying) is applied to the product and the bound water is removed by desorption (i.e. evaporation from liquid state to gaseous state, unlike sublimation which is evaporation from solid state to gaseous state), to eliminate the remaining water within the lyophilized tablet. Indeed, the presence of water within the product is often responsible for a rapid degradation of the active agent, resulting in low stability upon storage of the drug product.

This secondary drying step is generally operated at 40/+50°C and a pressure of around 50 to 100 µbars for a time of about 60 minutes or longer. The mean time in the lyophilizer varies generally from 60 to 90 minutes, but can be longer without any impact on the product.

The final lyophilized tablets are then thermosealed within their cavities, *i.e.,* blister packs, with aluminium foil at a temperature comprised between 120 and 160°C for a time of about 1 to 3 seconds.

One preferred category of homogenising agent according to the disclosure is the category of sugar esters.

Sugar esters are molecules resulting from the association of an osidic molecule with one or more fatty acid molecules by esterification. Sugar esters can be assimilated to a non-ionic surfactant exhibiting tensioactive properties, with the hydrophobic moiety comprising the fatty acid chain, while the hydrophilic moiety comprises the sugar molecule.

Suitable sugar esters of the disclosure include the esters of sorbitol and its derivatives, in particular sorbitan, and the esters of saccharose (also called sucrose), glucose, fructose and lactose. Sucrose and sorbitan esters are preferred.

Sucrose esters, in particular stearate esters of sucrose, are preferred sugar esters for use according to the present disclosure. More preferably the homogenising agents are preferably either mono or di-stearate esters of sucrose. These compounds are sold under the name of Sucroester®, which is a trade mark from Gattefosse. There are several grades of sucrose ester depending on the fatty acid linked. Preferred grades of sucrose ester for the disclosure are sucrose distearate (Sucroester® 7), sucrose mono/distearate (Sucroester® 11 ) and sucrose palmitate (Sucroester® 15).

Among the sugar esters suitable for the use according to the present disclosure, the polysorbates are also preferred. These compounds are derived from sorbitan esters, sorbitan being a molecule resulting from the dehydration of sorbitol.

Polysorbates are oily liquids with tensioactive properties that are derived from pegylated sorbitan and whose chemical name is polyoxyethylene sorbitan monolaurate, also called PEG sorbitan monolaurate and sold on the market under the trademark Tween®.

Polysorbates exist in different grades from Polysorbate 20 (polyoxyethylene (20) sorbitan monolaurate) to Polysorbate 80 (polyoxyethylene (80) sorbitan monolaurate). The number 20 following the *polyoxyethylene* part refers to the total number of oxyethylene -(CH₂CH₂O)- groups found in the molecule. The number following the *polysorbate* part is related to the type of fatty acid associated with the polyoxyethylene sorbitan part of the molecule. Monolaurate is indicated by 20, monopalmitate is indicated by 40, monostearate by 60 and monooleate by 80. For the present disclosure the grade Polysorbate 20, 60 and 80 are preferred polysorbate 60 (polyoxyethylene (20) sorbitan monostearate), being the most preferred.

One of the preferred homogenising agents of the present disclosure is a copolymer of polyvinylpyrrolidone and polyvinylvinylacetate, also called copovidone. Copovidone (also called copolyvidone) is the common name for a copolymer of 1-vinyl-2- pyrrolidone and vinyl acetate in a ratio of 6:4 by mass. Its chemical formula is (C₆H₉NO)ₙ x (C₄H₆O₂)ₙ. The acetate moiety is the hydrophobic moiety of the molecule while the pyrrolidone moiety provides its affinity for water. This compound exhibits some tensioactive properties and is soluble at more than 10 % in the following solvents: water, ethanol, glycerol and isopropanol. It is normally used as a dry binder in tabletting or as a granulating auxiliary or as a film-forming agent in the pharmaceutical industry. It is sold on the market under the trademark Kollidon® VA 64 (BASF)

Other possible homogenising agents for the use according to the present disclosure belong to the group of cellulose ethers polymers, including methylcellulose and hydroxypropyl methylcellulose. Such compounds present the polymeric backbone of cellulose, on which hydroxyl groups have been etherified with methyl molecules to form methoxy groups. The methyl groups representing the hydrophobic moiety while the hydroxyl group representing the hydrophilic moiety; these polymers also have tensioactive properties.

The preferred grades for use in the present disclosure are, methylcellulose polymer having from 27.5% to 31.5% of methoxyl groups and hydroxylpropyl methylcellulose polymer having a methoxyl content from 19% to 30% and a hydroxypropyl content of 7% to 12%. Such compounds are for example those sold under the name Methocel™ (provided by Colorcon Inc.)

The homogenising agent having tensioactive properties according to the present disclosure can also be a glyceride derivative molecule, i.e. esters of glycerol and fatty acids molecules.

Amongst these compounds may be cited in particular the mixtures of mono, di and triglycerides and mono and di ester of PEG, available commercially under the trademarks Labrafil® (oleoryl macrogol 6 glucosides), Labrafac® (medium-chain triglycerides, propylene glycol dicaprylocaprate), Labrasol® (caprylocapryl-macrogol 8 glycerides). Other suitable glyceride derivatives for the disclosure are glycerides with medium chain (Medium Chain triglycerides or MCT are a class of lipids in which three saturated fats are bound to a glycerol backbone, unlike Long Chain Triglycerides (LCTs) where each fat molecule is between 6 and 12 carbon in length) and partial glycerides. The term "partial glycerides" is employed in the present disclosure to designate esters of glycerol with fatty acids, whereby only a portion of the existing hydroxyl groups are esterified. In such compounds, at least one hydroxyl group within the glycerol ester remains free, contributing to the polar properties of the molecule.

Others categories of glyceride derivatives suitable for the present disclosure are mono esters of glycerol like, in particular glycerylmonostearate, or glycerylmono dicocoate. Therefore, some polyglyceride are also suitable as homogenising agents according to the disclosure, in particular glyceryl palmitostearate (for example as sold under the tradename Precirol® ATO5 from Gattefosse), polyglyceryl isostearate, polyglyceryl oleate or polyglyceryl palmitostearate.

Another category of suitable glyceride derivative usable as homogenising agent is glycerol formal, the term "glycerol formal" being employed to qualify the chemical compound also called methylidinoglycerol or Glicerinformal (sold under the trademark Sericosol® N), which is a mixture of 5-hydroxy-1,3-dioxane and 4-hydroxymethyl-1,3-dioxolane in a ratio of 60/40.

The glyceride derivatives cited above can eventually be used in mixtures as homogenising agents according to the present disclosure.

The homogenising agent having tensioactive properties according to the present disclosure can also be polyethylene glycol derivative. Poly(ethylene glycol)s, also known as PEGs, are an important type of polyether. PEG refers to an oligomer or a polymer of ethylene oxide having a molecular mass generally inferior to 20,000 g/mol. PEG polymers are prepared by polymerization of ethylene oxide and are commercially available over a wide range of molecular weights from 100 g/mol to 10,000,000 g/mol.

For the present disclosure, PEG derivatives have a molecular weight comprised between 100 and 8000, more preferably between about 4000 and 6000.

The PEG derivatives for use in the present disclosure are either pure polyethylene glycols compounds, preferably having a molecular weight comprised between 100 to 6000 or Polyethylene glycol molecules which have been esterified or etherified with hydrophobic molecules like fatty acids.

Amongst the PEG ethers that can be used according to the present disclosure, can be cited the Polyglycol isostearate which are dihydroxy ethers derived from the dehydration of two or more glycol molecules, preferred compounds having a PEG chain with a molecular weight varying from 100 g/mol to 1000g/mol, in particular polyglycol (300) isostearate and more particularly polyethylene 660, 12 hydroxystearate (sold under the trade mark Solutol® HS 15, BASF).

Others suitable polyethylene ethers for the use of the disclosure are polyglycol palmitostearate, preferably those having a PEG chain weighing from 400 to 6000.

Some oligomers composed of only two ethylene glycol units can also be mentioned as appropriate homogenising agents for the present disclosure. In particular di-ethylene glycol mono ethyl ether (sold under the trademark Transcutol®) is preferred.

The PEG derivative compounds belonging to the family of macrogol are also appropriate for the use object of the disclosure. Macrogol (Polyethylene glycol) is another name given linear polymers comprised of ethylene glycol molecules. One of the most preferred macrogol of the present disclosure is a polyglycol mono- and di-esters of 12 hydroxysteraric acid, sold under the trademark Solutol® HS 15 (provided by BASF). It is made of polyglycol mono- and di-esters of 12 hydroxysteraric acid (which is the lipophilic part) and of about 30% of free polyethylene glycol (which is the hydrophilic part)

Some commercial mixtures of polyethylene glycol ethers can also be used as homogenising agents having tensioactive properties for the disclosure. Mixtures derived from castor oil are preferred. More particularly, the polyoxyl hydrogenated castor oil compounds are of interest (sold under the trademark Cremophor®, BASF). The grade Cremophor® EL is also preferred. These compounds are prepared by reacting 35 moles of ethylene oxide with each mole of castor oil. The resulting product is a mixture whose major component is the material in which the hydroxyl groups of the castor oil triglyceride have ethoxylated with ethylene oxide to form polyethylene glycol ethers. Minor components are the polyethyelene glycol esters of ricinoleic acid, polyethyelene glycols and polyethyelene glycol ethers of glycerol .

The homogenising agent according to the present disclosure can also be a pharmaceutically acceptable surfactant having a high HLB (i.e., between about 15 and 20), alone or in combination with other homogenising agents according to the disclosure. Among these surfactants for use in the present disclosure the following ones are preferred: Sodium dioctyl sulfosuccinate, Calcium dioctyl sulfosuccinate, Sodium Lauryl sulfate, Magnesium Lauryl sulfate, Magnesium stearate, Sodium stearyl fumarate, Sodium oleate, Sodium benzoate and Sodium acetate.

The homogenising agent having tensioactive properties according to the present disclosure can also belong to the family of silicone emulsions, methylated siloxanes polymers (in particular Dimethicone, Cyclomethicone, Simethicone). This class of compounds (also called poly-dimethyl siloxane or polymerized siloxane) belongs to the group of compounds called silicon oils. Their chemical formula is (H₃C)₃SiO[Si(CH₃)2O]ₙSi(CH₃)₃, where n is the number of repeating monomer [SiO(CH₃)₂] units.

The homogenising agents can be included in concentrations varying from about 0.001 % to about 10 % by weight of the total dry weight of the tablet. Preferably, the amount of homogenising agent varies from 0.1 to 5 % by weight of the tablet and more preferably from 0.1 to 1 % by weight.

Depending on its physico-chemical characteristics and in particular its water-solubility, an active pharmaceutical ingredient, as described herein, can be employed in a conventional manner, e.g., under the form of a dry powder with micronized or nanonized particles or under the form of granulates. When the active agent not very soluble or even insoluble, it can be used as a liquid phase, i.e. dissolved within an organic solution which, in turn will form the organic phase of an oil-in-water (O/W) emulsion when put into contact with the final aqueous phase intended to be lyophilized.

An active pharmaceutical ingredient suitable for this disclosure can be any therapeutically active agent, and can be highly soluble, slightly soluble or even insoluble in water. In particular the formulation according to the present disclosure is appropriate for molecules presenting difficulties in formulation because of their low solubility, insolubility, instability and/or of bioavailability.

Any active pharmaceutical ingredient which can be employed as therapeutic agents can be used in the present disclosure, including: anti-inflammatory agents like piroxicam, ketoprofen, ibuprofen, flurbiprofen, or indomethacin, opiate compounds or other analgesics like morphine, hydrocodone, fentanyl, benzocaine, or paracetamol, antimicrobials and antibiotics agents like clarithromycine, amphotericin, azithromycin and erythromycin, antiasthmatics and medications intended for the treatment of allergies like loratadine, cetirizine, levocetirizine, and Bet v 1 (recombinant major allergen of birch pollen), antispasmodics, in particular phloroglucinol, antimigraine and neuroactive agents and in particular tiagabine, pregabalin, gabapentine, selegiline and wake promoting agents like adrafinil, modafinil, atomoxetine or armodafinil, antidepressant, sedatives, hypnotics and psychoactive agents like bupropion, venlafaxine hydrochloride, escitalopram, paroxetine hydrochloride, eszopiclone, clozapine, fluoxetin, fluvoxamine, sertraline hydrochloride, zolpidem or sulpiride, lipid lowering agents, in particular statines like simvastatin, rosuvastatin oratorvastatin calcium or ezetimibe or fibrates, in particular fenofibrate, anti emetics agents, in particular metopimazine and metoclopramide, anti diarrheic agents, in particular loperamide, antiulcer agents like omeprazole, aripiprazole and esomeprazole magnesium, anti obesity agents like orlistat, anticancer agents and anti-tumors agents in particular doxorubicin, arsenic trioxide, lestaurtinib and bendamustine, antithrombotics, antiplatelet and cardiovascular agents, in particular prasugrel, clopidogrel, diltiazem, dopexamine, Sch 530348, sildenafil, tadalafil, vardenafil hydrochloride and buflomedil, blood pressure lowering agents like verapamil and lisinopril.

The use of active pharmaceutical agents according to the disclosure can vary according to the active pharmaceutical ingredient used and its therapeutic activity and the resulting need to have a more or less highly concentrated dosage form. Therefore, the lyophilized tablets obtained according to the disclosure can contain from about 0.001 % to about 80 % of active pharmaceutical ingredient by weight of the total dry weight of the tablet. Preferably, the amount of active agent may vary from about 0.1 to about 50 % by weight of the tablet and more preferably from about 0.5 % to about 30% by weight.

The lyophilized tablets obtained according to the disclosure can advantageously contain two or more active pharmaceutical ingredients within the same drug unit.

The formulations of the present disclosure can also contain a diluent agent (also called a filler). Such substances are intended to mean pharmaceutically acceptable, preferably soluble materials and in some embodiments can improve the physical properties of the lyophilized form. These substances may be chosen from mannitol, lactose, glycine, sorbitol, glucose, maltodextrins, isomalt, cyclodextrins and their derivatives or mixtures thereof. Diluents/fillers may also be chosen from the oxides (e.g., magnesium oxide), carbonates (e.g., calcium carbonate), and phosphates (e.g., tricalcium phosphate). The diluents may constitute from 0.5 to 80% relative to the total dry mass of the freeze-dried product, the preferred quantity of diluent being from about 10 to about 50% of the dry mass.

The formulations of the present disclosure can also contain at least one binding agent, also called a binder. These excipients are used in the formulations of the present disclosure in a conventional manner so as to confer to the liquid phase to be lyophilized a rheological behaviour and a viscosity which are adapted to a good division of the product and to the maintenance of the various components in suspension. The purpose of these agents is to ensure the good homogeneity and stability of the liquid phase, which must have an acceptable flow to be easily processable at an industrial scale. Indeed, these substances may prevent the sedimentation of the particles contained within the liquid phase and ensure the correct texture of the final lyophilized product (for example a sufficient hardness to allow extrusion through a blister and an appropriate porosity to allow a quick and homogeneous disintegration of the tablet once in contact with saliva).

Such binders are understood to include any water-soluble or water-dispersible substance, which allows cohesion of the mass to be ensured, and is pharmaceutically acceptable and inert with respect to the active agent. These substances are in particular chosen from polypeptides such as gelatin or partly hydrolyzed gelatin, colloids, high molecular weight polysaccharides, high polymers capable of giving colloidal solutions, for example natural resins or gums (e.g. gum arabic, and gum tragacanth) synthetic or semi-synthetic gums (e.g. glycosylglucans, and xanthan gum), dextran, in particular grade called Dextran 20, 40 and 70, dextrin, alginates, in particular sodium alginate, pectinates, carboxymethylcellulose, water-dispersible starch derivatives, colloidal silicas, bentonites, or other support substances, such as polyvinyl alcohol, polyvinylpyrrolidone in particular grade called Povidone® 12, 17 or 30, acrylic polymers or copolymers, and even mixtures of such substances as those mentioned above. As a general rule, binders constitute from about 0.01 to about 30% relative to the dry mass of the freeze-dried product, preferably from about 0.5 to about 20%.

The oral dosage forms according to the present disclosure can contain advantageously one or more sweetening or taste modifying substances either alone or mixed together. Such excipients are employed conventionally to improve the taste of the active pharmaceutical ingredient or at least to mask the possible unpleasant taste of an active agent. In particular, commonly used sweetening and taste masking agents include: sucrose, glucose, xylose, sorbitol, sucralose, acesulfame, saccharin, saccharinates, cyclamates, aspartame, ammonium glycyrrhizinate or citric, ascorbic or tartaric acids. In general, any other substance normally used for taste modification in the pharmaceutical industry and which is compatible with the active agents are suitable for use within the present disclosure. The quantity of sweetening agents within solid oral lyophilized forms is generally from about 0.01 to about 5%, preferably about 0.05 to about 1 % by weight of the total dry weight of the composition.

The colouring agents and preserving agents are those usually employed in the pharmaceutical and food and cosmetic industry. Colouring agents suitable for the present disclosure are those commonly used for oral tablets in general and for oral lyophilized formulations and include, for example, according to the Food Drug and Cosmetic US federal code: amaranth, barley extract, caramel, carmine, carotene, chlorophyll-copper complex, aluminium lake, iron oxides, riboflavin, grape skin extract, titanium dioxide, erythrosine or methylene blue, etc.

In addition, the lyophilized product according to the present disclosure may contain other additives, in particular excipients used in order to prevent the breaking up of the lyophilized tablet. Among the agents which improve breaking up, use may be made of hydrophilic diluents or of disintegrating agents, especially some sugars (like levulose or sorbitol) or silica.

The present disclosure also covers oral lyophilizate compositions having an improved appearance obtained by the use of homogenising agents within the pharmaceutical formulation to be lyophilized. Such formulations contain at least one pharmaceutical active ingredient (API), one filler and /or one binder and at least one homogenising agent selected from the group consisting of: the copolymers of polyvinylpyrrolidone and polyvinylvinylacetate, the mixtures of mono, di and triglycerides and mono or diesters of poly ethylene glycol, the mono or diesters of sucrose, the polysorbates, and the methylcellulose derivatives, and mixtures of any of the foregoing.

The present disclosure also covers an oral lyophilizate having an improved appearance comprising at least a pharmaceutical active ingredient, a binder and/or a filler, and a homogenising agent, wherein the homogenising agent is a copolymer of 1-vinyl-2- pyrrolidone and vinyl acetate in a ratio of 6:4 by mass. Preferably, the amount of 1-vinyl-2- pyrrolidone and vinyl acetate in a ratio of 6:4 by mass in the oral lyophilizate represents from about 0.01 % to about 10 % by weight of the dry mass of the final dosage form. Preferably, copovidone is used from about 0.01 to about 2 %, more preferably between about 0.03 to about 0.10 % by weight of the dry mass of the final lyophilized tablet.

The present disclosure also covers an oral lyophilizate having an improved appearance comprising at least a pharmaceutical agent, a binder and/or a filler, and a homogenising agent, wherein the homogenising agent is polyoxyethylene (20) sorbitan monolaurate, monopalmitate, monostearate or monooleate (also called respectively polysorbate 20, 40, 60 and 80). More preferably, said homogenising agent is polyoxyethylene (20) sorbitan monostearate (polysorbate 60). Preferably, the amount of polysorbate 60 represents from about 0.03 to about 1 % by weight of the total dry weight of the oral lyophilizate, more preferably from about 0.05 to about 0.50 % by weight.

The present disclosure also covers an oral lyophilizate having an improved appearance comprising at least a pharmaceutical agent, a binder and or a filler, and a homogenising agent, wherein said homogenising agent is a sugar ester. Preferentially, the homogenising agent is a sucrose ester. More preferentially, the sucrose ester is a sucrose distearate (Sucroester® 7), a sucrose mono/distearate (Sucroester® 11) or a sucrose palmitate (Sucroester® 15). Preferably, the amount of sucrose ester by mass of the oral lyophilizate represents from about 0.01 to about 1 % by weight of the total dry weight of the oral lyophilizate, more preferably about 0.05 to about 0.50% by weight.

The present disclosure also covers an oral lyophilizate having an improved appearance comprising at least a pharmaceutical agent, a binder and or a filler, and a homogenising agent, wherein the homogenising agent is a methylcellulose polymer having from 27.5% to 31.5% of methoxyl groups and hydroxylpropylmethylcellulose polymer having methoxyl content from 19% to 30% and hydroxypropyl content of 7% to 12%. Preferably, the amount of methylcellulose or hydroxylpropyl methylcellulose compound by mass of the oral lyophilizate represents from about 0.01 to about 2 % by weight of the total dry weight of the oral lyophilizate, more preferably from about 0.05% to about 0.50 % by weight.

The present disclosure also covers an oral lyophilizate having an improved appearance comprising at least a pharmaceutical agent, a binder and/or a filler, and a homogenising agent, wherein the homogenising agent is oleoryl macrogol 6 glucosides (Labrafil®), or Labrafac® (medium-chain triglycerides, propylene glycol dicaprylocaprate), or caprylocapryl-macrogol 8 glycerides (Labrasol®). Preferably, the amount of mono, di and triglycerides and mono and di ester of PEG by mass of the oral lyophilizate represents from about 0.01 to about 1 % by weight of the total dry weight of the oral lyophilizate, more preferably from about 0.05% to about 0.5 % by weight.

The present disclosure also covers an oral lyophilizate having an improved appearance comprising at least a pharmaceutical agent, a binder and/or a filler, and a homogenising agent, wherein the homogenising agent is a polyethylene glycol derivative having a PEG chain with a molecular weight varying from 100 g/mol to 1000g/mol. Preferably, the homogenising agent is polyglycol (300) isostearate and more preferably polyethylene 660, 12 hydroxystearate (Solutol® HS 15). Preferably, the amount of polyethylene glycol derivative by mass of the oral lyophilizate represents from about 0.01 to about 2 % by weight of the total dry weight of the oral lyophilizate, more preferably from about 0.05 % to about 0.50 % by weight.

The present disclosure is of particular interest for the manufacture of all types of oral lyophilizate forms, as it allows the production of tablets having a flat, regular, smooth and homogenous surface and even coloration. The high quality appearance of the tablets may avoid the reluctance patients may have to ingest a dosage form of a rough and coarse aspect, which could be perceived to be an indication of low quality, low efficacy or even of an out-of-date product. These lyophilized dosage forms can also be used as drug products intended for paediatrics or for geriatrics. Finally, the lyophilized dosage forms are of great interest in the case of active substances which are insoluble, unstable, unacceptable in taste or of insufficient bioavailability.

### EXAMPLES

### Example 1

In this example, three lyophilized formulations (batches 1 to 3) using different concentrations of a glyceride derivative as a homogenising agent having tensioactive properties were manufactured.

The homogenising agent was caprylocapryl-macrogol 8 glycerides, sold under the trademark Labrasol® (Gattefosse).

In batch 1, no homogenising agent was included while in batches 2 and 3, the amount of homogenising agent represented respectively 0.14 and 0.7% of the total dry weight of the composition. The amounts of each excipient are presented in table 1.

The active pharmaceutical ingredient employed is phloroglucinol, an antispasmodic active pharmaceutical ingredient. Mannitol was used as filler and dextran was used as binder (under the grade Dextran 70) (Polymer of glucose with chain like structure and a molecular weight of 70000).

The manufacturing process was the same for each batch (with the proviso that no homogenising agent is added into the composition of batch 1) as described below.

In a mixer equipped with a vacuum system the powders of phloroglucinol, dextran 70 and mannitol were introduced and mixed together during about 10-20 minutes at a reduced pressure (1 00-300HPa) in order to remove air from powder.

In parallel, a solution of the homogenising agent: caprylocapryl-macrogol 8 glycerides (sold under Labrasol®) dispersed into water was prepared by mixing. Once a homogenous solution was obtained, it was added into the mixer equipped with vacuum system by aspiration to the powder mix and the whole mixture was then stirred during 20 to 90 minutes at reduced pressure (from 100 to 300 HPa) until a homogeneous suspension was obtained.

The final liquid phase, which was under the form of a suspension was then distributed into cavities of a preformed PVC blister.

The product was then freezed at a temperature of ―30°C during not less than 30 minutes (preferred duration time being from 30 to 60 minutes) in a lyophilizer Usifroid SMH 90 (with a total shelves surface= 0.90m₂).

Once the composition was completely congealed, it was freeze-dried at a temperature increasing from -30°C to +50°C during from 300 to 420 minutes at a pressure of 200 to 400 µbars.

Once this step was completed, a secondary drying step was operated at +50°C at 50 to 100 µbars during at least 60 minutes, preferably between 60 to 120 minutes, more preferably around 90 minutes.

Table 1 below describes the quantities of each component of the liquid phase and the appearance, disintegration time and dissolution time of the lyophilized tablets thus obtained.

Disintegration time is determined by measuring the time it took for a lyophilizate to completely disintegrate in a beaker of 200 ml of purified water at room temperature (15-25°C). One lyoc tablet is dropped in a beaker containing 200 ml of purified water and manually mix by means of a spatula, the disintegration is complete when the size of the particles is less than 1500 microns. The measurement was repeated in 5 separate experiments.

Dissolution time is determined by measuring the time necessary for all ingredients to be dissolved and the solution to become clear without any visible solid particles. The size of visible particles being superior to 125 microns, the dissolution is complete when all the particles are less then 125 microns.

The appearance of the tablets is evaluated by visual observation. If no alterations are visible at the surface of the tablet, the surface is qualified as "smooth", if some acceptable wrinkles or irregularities at the surface are present, the surface is qualified as "almost smooth". If large irregularities and/or domes are presents, the surface is qualified as irregular".

**Table 1**

| **Batches numbers** | **1** | | **2** | | **3** | |
|---|---|---|---|---|---|---|
| Composition | Mg/unit | % | Mg/unit | % | Mg/unit | % |
| Phloroglucinol | 62,25 | 8,9 | 62,25 | 8,9 | 62,25 | 8,9 |
| Dextran® 70 | 25 | 3,6 | 25 | 3,6 | 25 | 3,6 |
| Mannitol | 612,75 | 87,5 | 611,75 | 87,4 | 607,75 | 86,8 |
| Labrasol® (caprylocapryl-macrogol 8 glycerides) | 0 | 0 | 1 | 0,14 | 5 | 0,7 |
| Purified water disappearing after lyophilization | 500 | | 500 | | 500 | |
| Appearance | Surface : irregular | | Surface : almost smooth | | Surface : smooth | |
| | Sticks to the blister | | No sticking to the blister | | No sticking to the blister | |
| Disintegration | 1 ― 5 seconds | | 1 ― 5 seconds | | 5 ― 10 seconds | |
| Dissolution : 1 unit in 50ml purified water | 60 ― 90 seconds | | 60 ― 90 seconds | | 90 ― 150 seconds | |

It appears clearly from the results in table 1 that the method for preparing oral lyophilizate composition according to the present disclosure comprising the formation of a liquid phase by using a homogenising agent, clearly improves the physical appearance of the tablets obtained by such a method in comparison with control oral lyophilizate which does not comprise a homogenising agent.

Such results can be supported by the photographs N° 1 to 3 of batches 1 to 3 (Figure 1).

### Example 2

In this example, three lyophilized formulations (batches 4 to 6) were manufactured according to the same process described in example 1. Each formulation contained a different kind of homogenising agent having tensioactive properties according to the disclosure.

For all batches, the active pharmaceutical ingredient was phloroglucinol, the binder was dextran and the filler was mannitol, all these compounds were used in the same concentration for the three batches.

Batch 4: Macrogol 15 hydroxystearate (Solutol® HS 15) at 0.2 % of the dry weight of the final tablet.

Batch 5: Oleoryl macrogol 6 glucosides (a glyceride derivative sold under the trademark Labrafil®) at 0.2 % of the dry weight of the final tablet.

Batch 6: Polysorbate 60 (polyoxyethylene (20) sorbitan monostearate) at 0.2 % of the dry weight of the final tablet.

The hardness of the lyophilized solid dosage form obtained from this example is tested using a tablet hardness meter manufactured by Schleuniger Pharmatron. This is an electrically operated tablet tester that eliminates operator variability. The tests are performed with 10 tablets and the mean value is determined. Tablet hardness is represented by the force needed to crush the tablet (expressed in kiloponds (kp) or Newton (N) units). A larger number indicates a stronger tablet.

The appearance is evaluated by visual analysis according to the same protocol than used in example 1.

Table 2 described the quantities of each component of the liquid phase and the main characteristics of the lyophilized tablets thus obtained.

**Table 2**

| **Batches** | **4** | **5** | **6** |
|---|---|---|---|
| Composition | mg/unit | mg/unit | mg/unit |
| Phloroglucinol | 124.5 | 124.5 | 124.50 |
| Dextran 70 | 15.0 | 15.0 | 15.0 |
| Solutol® HS 15 | 0.5 (0.2%) | | |
| Labrafil® | | 0.5 (0.2%) | |
| Polysorbate 60 | | | 0.5 (0.2%) |
| Isomalt | 30.0 | 30.0 | 30.0 |
| Sucralose | 2.0 | 2.0 | 2.0 |
| Mannitol | 78.0 | 78.0 | 78.0 |
| Purified water disappearing after lyophilization | QS | QS | QS |
| Lyophilized drug product weight | 250 | 250 | 250 |

| **Drug product characteristics** | | | |
|---|---|---|---|
| Appearance | Smooth uniform surface | Smooth uniform surface | Smooth uniform surface |
| Hardness | 10-20 N | 10-20 N | 15-30 N |
| Disintegration time | 10-20 seconds | 10-20 seconds | 5-10 seconds |

The results exposed in table 2 show that the use of slight amounts of homogenising agents having tensioactive properties improves the physical appearance of the tablets obtained by such a process.

### Example 3

In this example, two comparative formulations (batches 7 and 8) of lyophilized products were manufactured at a semi-industrial scale (1000 tablets per batch).

Batch 8 did not contain any homogenising agent while batch 7 contained a copolymer of polyvinylpyrrolidone and polyvinylvinylacetate, also called copovidone, used as a homogenising agent according to the present disclosure. Copovidone used in this example is sold by BASF under the trademark Kollidon® VA 64. Copovidone was used in batch 7 at a concentration of 0.043% of the total dry weight of the final lyophilized tablets. With the exception of the presence of this homogenising agent, the two batches' compositions were strictly identical.

The active pharmaceutical ingredient was a very slightly water soluble anti-allergic agent: loratadine. The filler was mannitol and the binder was dextran 70. One sweetener (sucralose), a flavour agent (blackcurrant) and one preservative agent (citric acid) used to control the pH for stability improvement, have been advantageously added to these formulations.

The amounts of each component of batches 7 and 8 formulations are set up in Table 3.

**Table 3**

| **Batch number** | **7** | **8** |
|---|---|---|
| Batch size | 1000 lyocs | 1000 lyocs |
| Composition | mg/unit | mg/unit |
| Loratadine | 10.0 | 10.0 |
| Copovidone (Kollidon VA 64) | 0.1 (0.043%) | 0.0 |
| Dextran 70 | 6.0 | 6.0 |
| Citric acid | 0.5 | 0.5 |
| Flavor | 2.0 | 2.0 |
| Sucralose | 0.5 | 0.5 |
| Mannitol | 210.9 | 211.0 |
| Purified water | 130.0 | 130.0 |
| Lyophilized drug product weight | 230.0 | 230.0 |

The manufacturing process was the following: firstly, a suspension was prepared by mixing into water loratadine, mannitol, dextran, flavour agent, sucralose and citric acid, and the determined amount of copovidone for batch 7 only. The complete wetting of loratadine occurred in around 1 minute for batch 7 while in around 30 minutes for batch 8. The mixing of these components was performed during two hours.

Once a homogenous suspension was obtained, the distribution was performed in 360mg/unit blisters for batches 7 and 8.

The freezing step was operated in a conventional manner at -25°C for each batch.

Then, a primary drying was performed, for each batch from -30°C to + 30°C at 250 µbars during 300 minutes.

To ensure a complete drying, a secondary drying step was carried out at low pressure (10µ bars) on a first "ramp" *(i.e.* an increase of temperature of the shelves from 30°C to 45°C) with temperatures increasing from 30°C to 45°C during 5 to 15 minutes. The temperature was then kept at 45°C during 120 minutes, after which a decrease of temperature (second ramp) was applied from 45°C to 25°C during 10 to 20minutes. Then the temperature was hold at 25°C until the products were unloaded from the shelves.

The appearance is evaluated by visual analysis according to the same protocol than used in example 1.

The main characteristic of the lyophilized tablets obtained are summarized in Table 4 and photographs showing the appearance of tablets of each resulting batch are shown in Figure 2.

**Table 4**

| **Batch number** | **7** | **8** |
|---|---|---|
| Appearance | Smooth surface | Irregular surface |
| Disintegration (USP - SOTAX DT2) | 11 seconds | 11 seconds |
| Weight Uniformity | 237.4 mg SD:1.4% | 235.7 mg SD : 3.14 % |
| Hardness | 36.8 N | 20.7 N |
| Moisture | 0.58 % | 0.67 % |
| Friability after 50 rotations | 1.01 % | 1.53% |

It appears clearly from this example that the tablets manufactured according to the method of the present disclosure, comprising the formation of a liquid phase by the use of at least one homogenising agent, present a smooth surface while the absence of homogenising agent lead to tablets with an irregular surface. The use of homogenising agent, even at a very low quantity has also shown that the intrinsic properties of the lyophilized tablet are not altered by the use of copovidone but, to the contrary, improved. Indeed, tablets of batch 7 exhibit a higher hardness and a lower friability than those of batch 8 while conserving a quick disintegration time.

This is an important advantage of the disclosure as it renders the drug product less subject to alteration especially when unpacked by the patient. Therefore, the observed increasing of hardness was not responsible for a decrease of the disintegration speed which occurs frequently, at least for conventional tablets obtained by compression. The moisture content is also lower compared with tablet where no homogenising agents were used.

Th e hardness of the tablet obtained according to this example was determined in the same manner as described in example 2 above.

Moisture is determined by loss on drying (LOD) according to USP (section 731) water content was calculated by determining the loss of mass following the drying of at least 1 g of milled lyophilizate in an electrosensitive balance.

Disintegration time is determined by measuring the time it took for a lyophilizate to disintegrate in a beaker of 200 ml of purified water at room temperature (15-25°C). One lyoc tablet was dropped in a beaker containing 200 ml of purified water and manually mix by means of a spatula, the disintegration is complete when the size of the particles is less than 1500 microns. The measurement was repeated in 5 separate experiments.

Friability is using USP friabilator but the data is obtained after only 50 rotations instead of 100 rotations for conventional tablets.

### Example 4

In this example four, distinct lyophilized comparative formulations were manufactured, three of them according to the method of the present disclosure (batches 10, 11 and 12).

The ability of tablets not to stick to their blister packaging was compared.

Tablets were obtained either without any homogenising agent (batch 9) or with the use of 0.1 % by weight of a homogenising agent (batch 10, 11 and 12).

The composition of each batch in homogenising agent was the following:
> Batch 9: No homogenising agent
> Batch 10: 0.01 % caprylo capryl macrogol 8 glycerides (Labrasol®)
> Batch 11: 0.01 % oleoryl macrogol 6 glucosides (Labrafil®)
> Batch 12: 0.01 % Polysorbate 60 (polyoxyethylene (20) sorbitan monostearate)

The active pharmaceutical ingredient used was the slightly water soluble anti-inflammatory compound piroxicam which was used under a micronized form. The filler was lactose and a sweetener (sucralose) was added to each composition. The manufacturing process was identical to the one described in Example 3.

The amount of each component together with the main characteristics and appearance of the tablets thus obtained are summarized in Table 5.

**Table 5**

| **Batch number** | **9** | **10** | **11** | **12** |
|---|---|---|---|---|
| Composition | mg/unit | mg/unit | mg/unit | mg/unit |
| Piroxicam micronizedl | 10.0 | 10.0 | 10.0 | 10.0 |
| Povidone 30 | 20.0 | 20.0 | 20.0 | 20.0 |
| Labrasol | | 0.2 (0.1 %) | | -- |
| Labrafil | | | 0.2 (0.1 %) | -- |
| Polysorbate 60 | | | | 0.2 (0.1 %) |
| Sucralose | 1.0 | 1.0 | 1.0 | 1.0 |
| Lactose | 169.0 | 168.8 | 168.8 | 168.8 |
| Purified water disappearing after lyophilization | QS | QS | QS | QS |
| Lyophilized drug product weight | 200 | 200 | 200 | 200 |

| **Drug product characteristics** | | | | |
|---|---|---|---|---|
| Adherence to blister | Sticks to blister | No sticking to blister | No sticking to blister | No sticking to blister |
| Disintegration time | 2 - 5 seconds | 20-30 seconds | 10-20 seconds | 2 - 5 seconds |

This example shows that the use of very small quantities of a homogenising agent according to the disclosure minimizes the sticking of the tablets obtained to the blister in which they are manufactured in comparison with control tablets which do not comprise a homogenising agent. This allows to the tablets according to the disclosure to keep their original shape, structure and appearance even after having being removed from their package, thus contributing greatly to the final appearance of the lyophilized drug product.

### Example 5

In this example, fenofibrate, a very slightly water soluble product was employed as active pharmaceutical ingredient.

The homogenising agent according to the disclosure is Polysorbate 80 (polyoxyethylene (80) sorbitan monooleate), a sugar ester derivative.

Polyvinyl pyrrolidone (grade "Povidone 30", i.e. having a molecular weight of 30,000) was used as binder and mannitol as filler.

The manufacturing process is the same than described in Example 1 above.

The appearance is evaluated by visual analysis according to the same protocol than used in example 1.

Quantities of each component are summarized in the Table 6 below, along with the appearance and disintegration time of the lyophilized tablets thus obtained.

Figure 3 represent a photograph of such tablets, evidencing their uniform and smooth surface.

**Table 6**

| **Batch number** | **13** |
|---|---|
| Composition | mg/unit |
| Fenofibrate micronized | 67 |
| Povidone 30 | 50 |
| Polysorbate 80 | 5 (1.66%) |
| Mannitol | 178 |

| **Drug product characterization** | |
|---|---|
| Appearance | Smooth surface |
| Adherence to blister | No sticking to blister |
| Disintegration time | 5-10 seconds |

This example shows that a method according to the disclosure for manufacturing oral lyophilizate compositions, including the use of a small quantity of a homogenising agent (less than 2% by weight of the dry tablet) allow the tablet to exhibit a smooth and uniform surface while not modifying one of the main characteristics of such tablets: having a very short disintegration time.

This example also shows that the method according to the present disclosure allows formulating very slightly soluble products.

### Example 6

In this example, three distinct lyophilized comparative formulations without active pharmaceutical ingredient were manufactured, two of them according to the method of the present disclosure.

The appearance of tablets obtained without any homogenising agent (batch 16) versus the use of two distinct homogenising agents (batches 17 and 18) was compared.

The composition of each batch in homogenising agent according to the disclosure was the following:
> Batch 16: No homogenising agent
> Batch 17: 0.1 % sucroester 11 (Sucrose mono/distearate)
> Batch 18: 0.4% Methylcellulose (Methyl ether of cellulose)

The filler employed in this example was mannitol and the binder employed was dextran 70.

The manufacturing process was identical to the one described in Example 3.

The appearance is evaluated by visual analysis according to the same protocol than used in example 1.

The amount of each component together with the main characteristics and appearance of the tablets thus obtained are summarized in Table 7. Photographs of the tablets obtained for batches 16 and 18 are represented on Figures 4 and 5 respectively.

**Table 7**

| **Batch number** | **16** | **17** | **18** |
|---|---|---|---|
| Composition | mg/unit | mg/unit | mg/unit |
| Methylcellulose | -- | -- | 2 (0.4%) |
| Dextran 70 | 25 | 25 | 10 |
| Sucroester 11 | -- | 0.5 (0.1 %) | |
| Mannitol | 475 | 474.5 | 488 |
| Appearance | Irregular surface | Smooth uniform surface | Smooth uniform surface |
| Disintegration time | 10 - 15 seconds | 10 - 15 seconds | 15 - 20 seconds |
| Hardness (N) | 20-30 | 20-30 | 25-35 |

This example shows that the method according to the present disclosure comprising the formation of a liquid phase by the use of certain homogenising agents having tensioactive properties improves the visual appearance of lyophilized tablets. This phenomenon was observed even if the quantity of these homogenising agents is very low, here, e.g., less than about 0.5% by weight.

This example also shows that the method according to the present disclosure including the use of these homogenising agents improves the surface appearance but does not alter the main characteristics of the tablets (e.g., at least hardness and disintegration time) in comparison with control tablets which do not comprise a homogenising agent.

### Example 7

In this example, 3 coloured lyophilized formulations were manufactured according to the disclosure in order to analyse the effect of the homogenising agent on the uniformity of repartition of the colouring agent.

The homogenising agent employed was an ether of cellulose (cellulose methyl ether) sold under the trademark Methocel® A 15 (Dow Chemical) at a concentration of 1 mg/unit, representing for each batch 0.5% by weight of the total dry weight of the tablet.

These 3 compositions each contain a certain amount of colouring agent as described below:
Batch 19: Traces of Erythrosine (amount inferior to 0.0001 %)
Batch 20: 0.00025% by weight of Erythrosine
Batch 21: Traces of Methylene blue (amount inferior to 0.0001 %)

The filler used was mannitol and the binder was dextran 70, at same concentration for each batch. A sweetener (sucralose) and a flavouring agent (Strawberry) were also advantageously added to these compositions in order to improve the taste of the tablets.

The manufacturing process was identical to the one described in Example 3.

The appearance is evaluated on the basis of the uniform repartition of the colour at the surface of the tablet.

The amounts of each component together with the appearance and main characteristics of the tablets thus obtained are set up in Table 8, and photographs of the tablets obtained for batches 19 and 20 are reproduced on Figures 6 and 7.

**Table 8**

| **Batch number** | **19** | **20** | **21** |
|---|---|---|---|
| Composition | mg/unit | mg/unit | mg/unit |
| Methocel® A 15 Erythrosine | 1.00 (0.5% w/w) | 1.00 (0.5% w/w) | 1.00 (0.5% w/w) |
| Methylene blue | traces | 0.005 | -- |
| Dextran 70 | -- | -- | traces |
| Sucralose | 8.00 | 8.00 | 8.00 |
| Flavor | 0.20 | 0.20 | 0.20 |
| Mannitol | 1.00 | 1.00 | 1.00 |
| | 189.80 | 189.80 | 189.80 |
| Appearance | Uniform light pink coloured surface no sticking to blister | Uniform pink coloured surface No sticking to blister | Uniform surface light blue No sticking to blister |
| Disintegration time | 2 - 5 seconds | 2 - 5 seconds | 2 - 5 seconds |

The coloured tablets of this example show that the use of a small amount of a homogenising agent (here an ether of methylcellulose) does improve the surface appearance of the final tablet but is also responsible for the uniform repartition of a colouring agent into the whole tablet, even if this colouring agent is used at a very low concentration (traces, *i.e.:* amount inferior to 0.0001 %).

The above examples demonstrate the obvious advantages of the method of the present disclosure wherein the use of homogenising agents for the manufacture of lyophilized formulations allows an improvement in the uniformity and smoothness of the surface as well as the uniformity of the colouration and the anti-adhesive properties of each unit of the drug product.

## Claims

1. A method for preparing an oral lyophilizate composition comprising:
a) forming a liquid phase by using at least one homogenising agent having tensioactive properties, said liquid phase comprising at least an active pharmaceutical ingredient, a filler and/or a binding agent and a solvent,
b) lyophilizing said liquid phase to form the oral lyophilizate composition.

2. The method of claim 1, wherein the oral lyophilizate composition has an improved appearance relative to a control oral lyophilizate which does not comprise the homogenising agent.

3. The method of claim 1, wherein the homogenising agent is selected from the group consisting of sugar esters, copolymers of polyvinylpyrrolidone and polyvinyl actetate, cellulose ethers polymers, glyceride derivatives, polyethylene glycol derivatives, pharmaceutically acceptable surfactants having a HLB comprised between 15 and 20, and methylated siloxanes polymers

4. The method of claim 1, wherein the homogenising agent is a sugar ester belonging to the group selected from sucrose esters and sorbitan esters.

5. The method of claim 1 wherein the homogenising agent is a copolymer of 1-vinyl-2- pyrrolidone and vinyl acetate in a ratio of 6:4 by mass.

6. The method of claim 1 wherein the homogenising agent is a methylcellulose polymer having from 27.5% to 31.5% of methoxyl groups or an hydroxylpropylmethylcellulose polymer havinga methoxyl content from 19% to 30% and hydroxypropyl content of 7% to 12%.

7. The method of claim 1 wherein the homogenising agent is a glyceride derivative selected from the group consisting of mixtures of mono, di and triglycerides and mono and di esters of PEG, medium chain glycerides, glyceryl monostearate, glyceryl mono dicocoate, partial glycerides, polyglyceryl isostearate, oleate or palmitostearate, and mixtures of any of the foregoing.

8. The method of claim 1 wherein the homogenising agent is a polyethylene glycol derivative selected from the group consisting of polyethylene glycol having a molecular weight between about 100 to about 6000, polyoxyl hydrogenated castor oils and polyglycol ethers having a PEG chain with a molecular weight varying fromabout 100 g/mol to about 1000g/mol.

9. The method of claim 1 wherein the homogenising agent is a pharmaceutically acceptable surfactant having a HLB between about 15 and about 20 selected from the group consisting of sodium dioctyl sulfosuccinate, calcium dioctyl sulfosuccinate, sodium lauryl sulfate, magnesium lauryl sulfate, magnesium stearate, sodium stearyl fumarate, sodium oleate, sodium benzoate and sodium acetate.

10. The method of claim 1 wherein the homogenising agent represents from about 0.001 % to about 10 % by weight of the total dry weight of said oral lyophilizate composition.

11. The method of claim 10 wherein the homogenising agent represents from about 0.1 to about 5 % by weight of the total dry weight of the oral lyophilizate composition.

12. The method of claim 10 wherein the homogenising agent represents from about 0.1 to about 1 % by weight of the total dry weight of the oral lyophilizate composition.

13. A process for the manufacture of the oral lyophilizate composition comprising:
a. Preparing a liquid phase containing at least the active(s) pharmaceutical ingredient(s), a filler an/or a binding agent and a homogenising agent;
b. Stirring the liquid phase until a homogenous mixture is obtained;
c. Distributing the liquid preparation into preformed blisters or moulds
d. Freezing the resulting mixture between -20° to -50°C
e. Freeze-drying the congealed mixture
f. Optionally performing a secondary drying of the final mixture obtained

14. An oral lyophilizate composition having an improved appearance relative to a control oral lyophilizate which does not comprise the homogenising agent comprising: at least an active pharmaceutical ingredient, a filler and/ or a binding agent and a homogenising agent, wherein said homogenising agent is selected from the group consisting of copolymers of polyvinylpyrrolidone and polyvinylvinylacetate, sucrose esters, methylcellulose or hydroxylpropyl methylcellulose polymers, mixtures of mono, di and triglycerides and mono or diesters of poly ethylene glycol, polyethylene glycol derivatives having a PEG chain with a molecular weight varying from about 100 g/mol to about 1000g/mol, and mixtures of any of the foregoing.

15. An oral lyophilizate composition having an improved appearance relative to a control oral lyophilizate which does not comprise the homogenising agent comprising: an active pharmaceutical ingredient, a filler and/or a binding agent and a homogenising agent, wherein said homogenising agent is selected from the group consisting of a copolymer of 1-vinyl-2- pyrrolidone and vinyl acetate in a ratio of 6:4 by mass, sucrose distearate, mixtures of sucrose mono and distearate, sucrose palmitate, polyglycol (300) isostearate, polyethylene 660, 12 hydroxystearate, oleoryl macrogol 6 glucosides, glyceryl palmitostearate, medium chain triglycerides, propylene glycol dicaprylocaprate, caprylocapryl-macrogol 8 glycerides, methylcellulose polymer having from 27.5% to 31.5% of methoxyl groups and hydroxylpropyl methylcellulose polymer having a methoxyl content from 19% to 30% and a hydroxypropyl content of 7% to 12%, and mixtures of any of the foregoing.

16. An oral lyophilizate composition according to claim 15 wherein said active pharmaceutical ingredient is selected from the group consisting of: piroxicam, ketoprofen, ibuprofen, flurbiprofen, indomethacin, morphine, hydrocodone, fentanyl, benzocaine, paracetamol, clarithromycine, amphotericin, azithromycin and erythromycin, loratadine, cetirizine, levocetirizine and bet v 1, phloroglucinol, tiagabine, pregabalin, gabapentine, selegiline, adrafinil, modafinil, armodafinil, atomoxetine, bupropion, venlafaxine hydrochloride, escitalopram, paroxetine hydrochloride, eszopiclone, clozapine, fluoxetin, fluvoxamine, sertraline hydrochloride, zolpidem, sulpiride, simvastatin, rosuvastatin or atorvastatin calcium, ezetimibe, fenofibrate, metopimazine, metoclopramide, loperamide, omeprazole, aripiprazole, esomeprazole magnesium, orlistat, doxorubicin, arsenic trioxide, lestaurtinib, bendamustine, prasugrel, clopidogrel, diltiazem, dopexamine, sildenafil, tadalafil, vardenafil hydrochloride, buflomedil, verapamil and lisinopril.
